**Europäisches Patentamt**

**European Patent Office**

**Office européen des brevets**

(19)

(11) **EP 1 484 586 A1**

(12) ## EUROPÄISCHE PATENTANMELDUNG

(43) Veröffentlichungstag:
**08.12.2004 Patentblatt 2004/50**

(51) Int Cl.[7]: **G01G 17/00**, G01G 9/00,
G01N 22/04

(21) Anmeldenummer: **03012566.0**

(22) Anmeldetag: **03.06.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(71) Anmelder: **BOEHRINGER INGELHEIM PHARMA GMBH & CO. KG**
**55216 Ingelheim am Rhein (DE)**

(72) Erfinder: **Die Erfindernennung liegt noch nicht vor**

(54) **Messeinrichtung zur zerstörungsfreien Bestimmung der Einwaage in Kapseln**

(57) Die Erfindung betrifft ein Verfahren zur zerstörungsfreien Nettoverwiegung von Kapseln mit Hilfe von Mikrowellen.

Fig 2: Skizze zur Verbindung von Mikrowellendetektor und Wiegezellen

**EP 1 484 586 A1**

**Beschreibung**

Die Erfindung betrifft ein Verfahren zur zerstörungsfreien Nettoverwiegung von Kapseln mit Hilfe von Mikrowellen.

Hintergrund der Erfindung

In vielen industriellen Herstellprozessen ist die Kenntnis des Nettogewichts verpackter Ware (z.B. Schüttgüter, Lebensmittel usw.) von besonderer Bedeutung. Wenn das Taragewicht gegenüber der Einwaage vernachlässigbar klein ist, so genügt in der Regel eine Bruttoverwiegung der verpackten Ware. Sind die Gebinde jedoch sehr klein, wie z.B. bei Arzneimittelwirkstoffe enthaltenden pharmazeutischen Formulierungen, wie beispielsweise Kapseln, so ist die Relation zwischen Oberfläche und Volumen stark zugunsten der Oberfläche verschoben, und das Taragewicht überschreitet die Einwaage leicht um ein Mehrfaches. In solchen Fällen sind die Schwankungen des Taragewichts nicht mehr zu ignorieren. Die Einwaage muß daher entweder durch direkte Netto-Verwiegung des Inhalts bestimmt werden oder durch Verwiegung des Meßobjektes vor und nach der Verpackung (Tara- und Bruttoverwiegung).

Naturgemäß wird eine Nettoverwiegung stets auf der Abfüll- oder Verpackungsmaschine erfolgen, wo die eigentliche Dosierung stattfindet. Gleiches gilt aber auch für die Brutto/Tara-Verwiegung, wenn mit hohen Produktionsraten gearbeitet wird, denn andernfalls wäre die richtige Zuordnung zwischen Tara- und Bruttomeßwerten der einzelnen Meßstücke nur schwer oder nur mit hohem Aufwand zu gewährleisten.

Werden bei gleichzeitig geringen Einwaagen im mg-Bereich hohe Produktionsraten (z.B. 100000 Stück/Stunde und mehr) gefordert, so ergeben sich extreme Präzisions- und Schnelligkeitsanforderungen an die Meßapparatur. Im Stand der Technik sind beispielsweise Online-Brutto-Tara-Verwiegesysteme auf Kapselfüllmaschinen bekannt, bei denen ein kapazitives Meßsystem zum Einsatz gelangt. Dort wird das Meßgut vor und nach der Befüllung durch einen Kondensator geführt, aus dessen Kapazitätsveränderung man auf die jeweils eingebrachte Masse zurückrechnet. Solche Verfahren sind jedoch erfahrungsgemäß nur für Einwaagen größer als 50 mg spezifiziert. Moderne gravimetrische Wiegezellen erfüllen deutlich höhere Genauigkeitsspezifikationen. Sie arbeiten jedoch mit einer nur geringen Geschwindigkeit von einigen wenigen Kapseln pro Sekunde. Bei entsprechender Parallelschaltung solcher Wiegezellen sind zwar grundsätzlich höhere Durchsatzgeschwindigkeiten erreichbar, allerdings unterliegen gravimetrische Wiegezellen der Beschränkung, sehr empfindlich auf Vibrationen zu reagieren und im getakteten statt im kontinuierlichen Modus betrieben werden zu müssen. Dadurch sind gravimetrische Wiegezellen auf kontinuierlich arbeitenden Kapselfüllmaschinen nur sehr bedingt einsetzbar.

Es resultiert also ein Bedarf nach einer Verwiegemethode, die - von der Abfüllmaschine losgelöst als Standalone-Lösung fungierend - den Inhalt fertig befüllter und verschlossener Kapsel zu bestimmen in der Lage ist.

Beschreibung der Erfindung

Die vorstehend genannte Aufgabe wird durch das erfindungsgemäße Verfahren gelöst. Die Erfindung betrifft ein Verfahren zur zerstörungsfreien Nettoverwiegung von Kapseln mit Hilfe von Mikrowellen, bei dem in einem ersten Verfahrensschritt durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Messprobe in Wirkverbindung stehenden Applikators ist, dadurch gekennzeichnet, dass mit dem Resonator eine Materialprobe so in Verbindung gebracht wird, dass das elektrische Feld des Resonators beim Übergang in das Material der Probe allgemein parallel zur Oberfläche verläuft und dass aus den primären Messgrössen b(0) und f(0) die Messsignale

$$F = f(L) - f(0) \text{ bzw. } B = b(0) - b(L)$$

erhalten werden, wobei $F$ und $B$ für die Mikrowellenmesssignale der Verstimmung und Verbreiterung der Resonanz stehen, mit $f(L)$ und $b(L)$ gleich konstante materialabhängige Bezugsgrößen,
und bei dem in einem zweiten Schritt eine gravimetrische Bruttoverwiegung zur Bestimmung des Bruttowiegesignals

$$C = m_T + m_N,$$

in dem die Grösse $m_T$ für die Masse des leeren Gebindes und die Grösse $m_N$ für die zu bestimmende Nettomasse steht, durchgeführt wird, und abschließend aus den erhaltenen Messsignalen *F, B* und *C* die zu bestimmende Nettomasse $m_N$ gemäß nachfolgender Relation

$$m_N = \alpha A + \beta B + \gamma C + \delta$$

rechnerisch bestimmt wird, wobei die Koeffizienten $\alpha$, $\beta$, $\gamma$ und $\delta$ zuvor über Kalibrierung des Messsystems durch lineare Regression aus einer Meßreihe mit Referenzproben bekannter Einwaage bestimmt wurden.

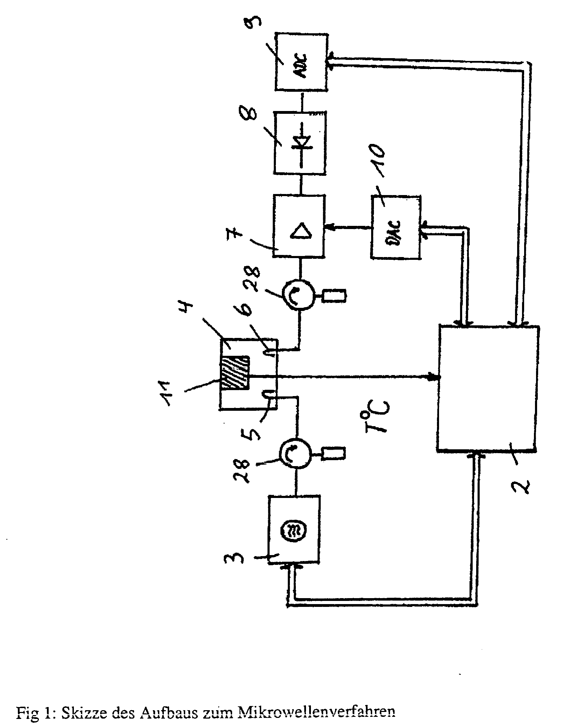

Zur Bestimmung der Meßsignale *F* und *B* können Vorrichtungen, wie sie beispielsweise in der EP 468023 A offenbart werden, zum Einsatz gelangen. Figur 1 offenbart den schematischen Aufbau einer solchen Vorrichtung.

Diese Vorrichtung ist gekennzeichnet durch einen von einem Prozessor (2) digital abstimmbaren Mikrowellengenerator (3) mit variabler Frequenz, der mit einer Ankopplungssonde (5) verbunden ist, die in einem Applikator (4) zur Messung der Meßsignale *F* und *B* einer Probe (11) angeordnet ist, der eine weitere Ankopplungssonde (6) aufweist, die über einen Mikrowellen-Verstärker bzw. -Abschwächer (7) mit einer Detektor-Diode (8) verbunden ist, deren Signalausgang mit dem Prozessor (2) verbunden ist.

Zur Bestimmung des Bruttowiegesignals C können gravimetrische Wiegezellen zum Einsatz gelangen, die das Gewicht mit einer Standardabweichung von +/- 0.1 mg bestimmen.

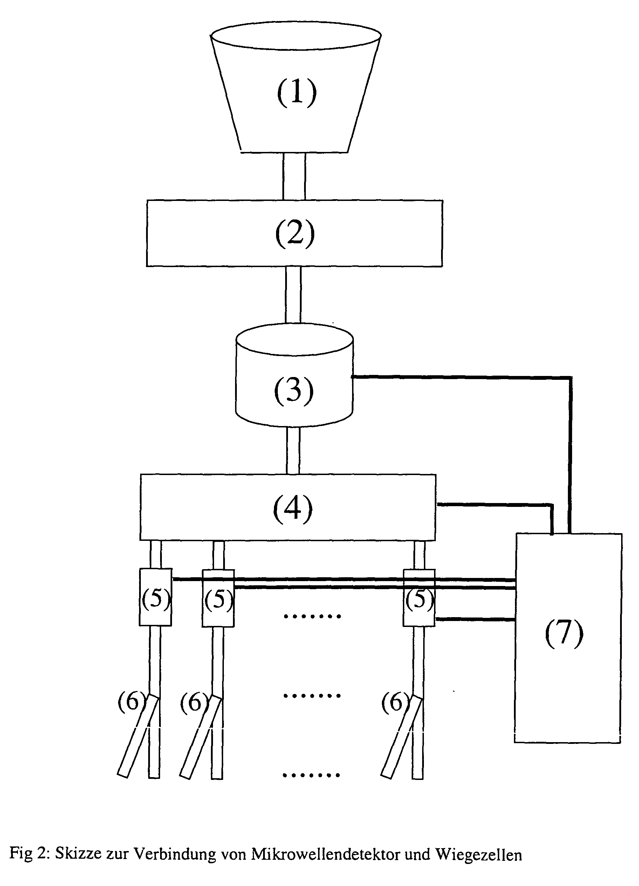

Figur 2 offenbart den schematischen Aufbau der Ankopplung des Mikrowellenvervahrens an die Bruttoverwiegung. Mit Hilfe einer Vereinzelungseinheit (2) werden die befüllten und verschlossenen Kapseln aus einem Trichter (1) seriell und/oder einzeln durch den Mikrowellenresonator (3) geführt. Eine sich daran anschließende Verteilereinheit (4) wie sie in heutigen Kapselwiegemaschinen eingesetzt wird, parallelisiert den Transport und verteilt die Kapseln auf mehrere Spuren, in denen sich die gravimetrischen Wiegezellen (5) befinden. Eine computergestützte Datenerfassungseinheit (7), die mit den Einheiten (3), (4) und (5) verbunden ist, sorgt für die richtige Zuordnung der Meßwerte zu den einzelnen Kapseln und führt die Auswertung durch. Kapseln mit fehlgewichtiger Einwaage werden durch eine Weiche (6) ausgeschieden.

Unter Mikrowellenresonator (3) wird dabei eine Vorrichtung gemäß Figur 1 verstanden.

Dieses Verfahren ist geeignet für Einwaagen bis hinunter in den mg-Bereich. Es arbeitet mit hoher Geschwindigkeit und ist selbst dann einsetzbar, wenn das Gewicht der Verpackung bzw. der leeren Kapsel die Einwaage des Kapselinhalts weit überschreitet.

Der besondere Nutzen des Verfahrens besteht darin, dass die erforderliche Messung mit hoher Geschwindigkeit und Präzision durchführen zu können, so daß auch noch Einwaagen im mg-Bereich verarbeitet werden können. Schwankungen in der Produkt- oder Packungsmittelfeuchte werden durch das erfindungsgemäße Verfahren ebenfalls kompensiert.

Die Auswertung der durch das erfindungsgemäße Verfahren erhaltenen Datensätze erfolgt wie nachstehend beschrieben.

Die Massen der Verpackung (Tara) und des Meßgutes (Netto) werden mit $m_T$, $m_N$ bezeichnet. Entweder die Verpackung oder der Inhalt dürfen wasserhaltig sein. Die entweder in der Leerkapsel oder im Füllgut enthaltene Wassermasse wird nachstehend mit $m_{H2O}$ bezeichnet.

Die durch die Mikrowellenmeßmethode bestimmten beiden Meßsignale für Verstimmung und Verbreiterung der Resonanz werden nachstehend mit F = *f (L)* - *f (0)* bzw.

B = *b*(0) - *b*(L) bezeichnet und das Resultat der Bruttoverwiegung mit *C*.

Die Verstimmung des Mikrowellenresonators F als auch die Verbreiterung der Resonanz B sind proportional zur Masse der jeweils in den Resonator eingebrachten Probe. Mithin können die beiden Mikrowellenmeßsignale in der folgenden Form dargestellt werden

$$F = f_T m_T + f_{H2O} m_{H2O} + f_N m_N, \tag{1}$$

$$B = b_T m_T + b_{H2O} m_{H2O} + b_N m_N \tag{2}$$

Hierbei stehen die Koeffizienten $f_T$, $f_{H2O}$, $f_N$ und $b_T$, $b_{H2O}$, $b_N$ für die jeweils substanzspezifischen Proportionalitätskonstanten.

Da die Mikrowellendämpfung in erster Linie vom Wassergehalt des Meßgutes abhängt, wird das Meßsignal B im Wesentlichen durch den Term $b_{H2O} m_{H2O}$ in Gleichung (2) bestimmt. Schwankungen in entweder der Produkt- oder der Packungsmittelfeuchte werden also durch Berücksichtigung des Meßwertes B kompensiert.

Für das Bruttowiegesignal gilt

$$C = m_T + m_N. \tag{3}$$

Nach Inversion des linearen Gleichungssystems (1),(2),3) und Auflösung nach der gesuchten Nettoeinwaage erhält man $m_N$ als Linearkombination der erhobenen Meßdaten $A$, $B$, und $C$ in der Form

$$m_N = \alpha A + \beta B + \gamma C + \delta. \tag{4}$$

Zur Kalibrierung werden die Koeffizienten $\alpha$, $\beta$, $\gamma$, $\delta$ in Gleichung (4) durch lineare Regression aus einer Meßreihe mit Referenzproben bekannter Einwaage bestimmt. Der Koeffizient $\delta$ trägt hier einer möglichen Verschiebung der Kalibrierung durch andere Einflußfaktoren (z.B. Temperatur) Rechnung.

Da die Mikrowellenverstimmung $F$ stets zur jeweiligen Dielektrizitätskonstanten (DK) der in den Resonator eingebrachten Materialen proportional ist, ist das Funktionsprinzip der vorgeschlagenen Apparatur gewährleistet, wenn die DK's von Packmittel und Inhalt hinreichend verschieden sind.

Unter Kapseln werden im Rahmen der vorliegenden Erfindung geschlossene Kapseln verstanden, die durch einen Gehalt an einem Pulver gekennzeichnet sind.

Das Material, aus welchem diese Kapseln bestehen ist erfindungsgemäß ausgewählt aus der Gruppe bestehend aus Gelatine, Cellulosederivaten, Stärke, Stärkederivaten, Chitosan und synthetischen Kunststoffen. Wird Gelatine als Kapselmaterial verwendet, so kann diese im Gemisch mit anderen Zusätzen ausgewählt aus der Gruppe bestehend aus Polyethylenglycol (PEG), bevorzugt PEG 3350, Glycerol, Sorbitol, Propylenglycol, PEO-PPO-Blockcopolymeren und anderen Polyalkoholen und Polyethern zum Einsatz kommen. Besonders bevorzugt wird im Rahmen der vorliegenden Erfindung Gelatine im Gemisch mit PEG, bevorzugt PEG 3350, verwendet. Besonders bevorzugt enthält eine erfindungsgemäße Gelatine-Kapsel PEG in einem Anteil von 1-10% (Gew-%), bevorzugt 3-8 %. Besonders bevorzugte Gelatine-Kapseln enthalten PEG in einem Anteil von 4-6%, wobei ein PEG-Anteil von etwa 5% erfindungsgemäß höchstbevorzugt ist.

Werden Cellulosederivate als Kapselmaterial verwendet, so ist die Verwendung von Hydroxypropylmethylcellulose, Hydroxypropylcellulose, Methylcellulose, Hydroxymethylcellulose und Hydroxyethylcellulose bevorzugt. Besonders bevorzugt wird in diesem Fall Hydroxypropylmethylcellulose (HPMC), besonders bevorzugt HPMC 2910 als Kapselmaterial eingesetzt. Werden als Kapselmaterial synthetische Kunststoffe eingesetzt, so sind diese erfindungsgemäß bevorzugt ausgewählt aus der Gruppe bestehend aus Polyethylen Polycarbonat, Polyester, Polypropylen und Polyethylenterephthalat. Besonders bevorzugt sind als synthetische Kunststoffmaterialien für die erfindungsgemäßen Inhalationskapseln Polyethylen, Polycarbonat oder Polyethylenterephthalat. Wird Polyethylen als eines der erfindungsgemäß besonders bevorzugten Kapselmaterialien verwendet, gelangt vorzugsweise Polyethylen mit einer Dichte zwischen 900 und 1000 kg/m$^3$, bevorzugt von 940 - 980 kg/m$^3$, besonders bevorzugt von 960 kg/m$^3$ (high-density Polyethylen) zur Anwendung.

Die Herstellung der leeren Kapseln kann nach im Stand der Technik bekannten Vorgehensweisen erfolgen. Beispielsweise seien als mögliche Herstellungsverfahren die im Stand der Technik bekannten Prozesse des Tauchverfahrens, Blasdruckverfahrens, Spritzgußverfahrens, Extrusionsverfahrens und Taufziehverfahrens genannt.

Das erfindungsgemäße Verfahren zur Nettoverwiegung von Kapseln zielt ab auf die Bestimmung der genauen Einwaage bzw. Masse $m_N$ des in den Kapseln enthaltenen Pulvers. Bei diesem Pulver handelt es sich vorzugsweise um ein für die Inhalation vorgesehenes Pulver, welches neben einem Wirkstoff einen pharmazeutisch verträglichen Hilfsstoff enthält.

Als pharmazeutisch verträgliche Hilfsstoffe seien beispielsweise genannt Monosaccharide (z.B. Glucose oder Arabinose), Disaccharide (z.B. Lactose, Saccharose, Maltose, Trehalose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Als Wirkstoffe werden im Rahmen der vorliegenden Erfindung Verbindungen verstanden, die bevorzugt ausgewählt sind aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

Als Anticholinergika kommen bevorzugt Verbindungen ausgewählt aus der Gruppe der Tiotropiumsalze, Ipratropiumsalze, Oxitropiumsalze,

Salze der aus der WO 02/32899 bekannten Verbindungen

N-Methyl-2,2-Diphenylpropionsäuretropenolester,

N-Methyl-2,2-Diphenylpropionsäurescopinester,

N-Methyl-2-Fluor-2,2-Diphenylessigsäurescopinester und

N-Methyl-2-Fluor-2,2-Diphenylessigsäuretropenolester

sowie Salze der aus der WO 02/32899 bekannten Verbindungen

N-Methyl-3,3',4,4'-Tetrafluorbenzilsäuretropenolester,

N-Methyl-3,3',4,4'-Tetrafluorbenzilsäurescopinester;

N-Methyl-4,4'-Dichlorbenzilsäurescopinester,

N-Methyl- 4,4'-Difluorbenzilsäurescopinester,

N-Methyl-3,3'-Difluorbenzilsäuretropenolester,

N-Methyl- 3,3'-Difluorbenzilsäurescopinester und

N-Ethyl-4,4'-Difluorbenzilsäuretropenolester gegebenenfalls in Form ihrer Hydrate und Solvate, in Betracht.

Unter Salzen sind dabei diejenigen Verbindungen zu verstehen, die neben den vorstehend genannten Kationen als Gegenion ein einfach negativ geladenes Anion ausgewählt aus der Gruppe Chlorid, Bromid und Methansulfonat enthalten.

Besonders bevorzugt kommen als Wirkstoffe im Rahmen der vorliegenden Erfindung die Bromide oder Methansulfonate der vorstehend genannten Strukturen in Betracht.

Von herausragendem Interesse sind im Rahmen der vorliegenden Erfindung beispielsweise Anticholinergika Tiotropiumbromid, Ipratropiumbromid, Oxitropiumbromid, 2,2-Diphenylpropionsäuretropenolester-methobromid, 2,2-Diphenylpropionsäurescopinester-methobromid, 2-Fluor-2,2-Diphenylessigsäurescopinester-methobromid, 2-Fluor-2,2-Diphenylessigsäuretropenolester-methobromid, 3,3',4,4'-Tetrafluorbenzilsäuretropenolester-methobromid, 3,3',4,4'-Tetrafluorbenzilsäurescopinester-methobromid; 4,4'-Dichlorbenzilsäurescopinestermethobromid, 4,4'-Difluorbenzilsäurescopinester-methobromid, 3,3'-Difluorbenzilsäuretropenolester-methobromid, 3,3'-Difluorbenzilsäurescopinester-methobromid und 4,4'-Difluorbenzilsäuretropenolester-ethylbromid woebi der Tiotropiumbromid, dem Ipratropiumbromid, dem 2,2-Diphenylpropionsäuretropenolester-methobromid, dem 2,2-Diphenylpropionsäurescopinester-methobromid, dem 2-Fluor-2,2-Diphenylessigsäurescopinester-methobromid sowie dem 2-Fluor-2,2-Diphenylessigsäuretropenolester-methobromid besondere Bedeutung zukommen.

Von herausragender Bedeutung ist hierbei das Tiotropiumbromid, besonders bevorzugt in Form seines aus der WO 02/30928 bekannten kristallinen Monohydrats.

Als Betamimetika kommen erfindungsgemäß bevorzugt diejenigen Verbindungen in Betracht, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salbutamol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-S-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-nbutyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1, 2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-tert.butylamino)ethanol und 1-(4-Ethoxycarbonylamino-3-cyano-5-fluorophenyl)-2-(tert.butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe zur Anwendung, die ausgewählt sind aus der Gruppe bestehend aus Fenoterol, Formoterol, Salmeterol, Salbutamol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-nbutyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1, 2,4-triazol-3-yl]-2-methyl-2-butylamino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.

Beispeilsweise sind die Säureadditionssalze der Betamimetika ausgewählt aus der Gruppe bestehend aus Hydrochlorid, Hydrobromid, Sulfat, Phosphat, Fumarat Methansulfonat und Xinafoat.

Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat. Besonders bevorzugt sind die Salze im Falle des Salmeterols ausgewählt aus Hydrochlorid, Sulfat und Xinafoat, von denen das Xinafoat besonders bevorzugt ist.

Im Rahmen der vorliegenden Erfindung werden unter Corticosteroiden, die erfindungsgemäß zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure, Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Die nachfolgenden Ausführungsbeispiele dienen der weitergehenden Erläuterung der Erfindung. Die folgenden Meßresultate wurden an einem Ensemble von 25 mit einem Tiotropiumbromid-haltigen Inhalationspulver befüllten Kapseln erzielt.

Die Mikrowellenmeßwerte F und B wurden mit Hilfe einer durch die Firma TEWS Elektronik (Sperberhorst 10, D-22459 Hamburg) konstruierten und gelieferten Mikrowellen-Feuchtemeßanlage MW3010, der Mikrowellenauswerteelektronik MW3010 und dem Resonator E83/8 gemessen. Die Bruttoverwiegungen zur Ermittlung des Meßwertes C wurden mit Hilfe einer Analysenwaage AX105 der Firma Mettler Toledo GmbH (Im Langacher, CH-8606 Greifensee, Schweiz) bei der reduzierten Ablesegenauigkeit von 0.1mg vorgenommen.

Zur Kalibrierung der Apparatur und zur Bewertung der erzielbaren Genauigkeit des Verfahrens war es notwendig, die wahren Einwaagen $m_N$ der 25 Kapseln genau zu kennen. Dazu wurden die Kapseln manuell durch einen Handpulverstechheber befüllt. Durch jeweilige Verwiegung der einzelnen Kapseln vor und nach der Befüllung auf der o.g. Mettler Analysewaage bei einer Ablesegenauigkeit von 0.01mg wurde das Nettofüllgewicht ermittelt.

Tabelle 1 faßt alle Meß- und Einwaagewerte zusammen. Die ersten 15 Proben wurden zur Gerätekalibrierung verwendet. Zu diesem Zweck wurden die Koeffizienten $\alpha$, $\beta$, $\gamma$, $\delta$ aus Gleichung(4) durch lineare Regression der Meßwerte A,B, C mit den bekannten Einwaagewerten $m_N$ berechnet.

Als Zusammengang zwischen Meßsignal und Einwaage wurde daraus erhalten

$$m_N = -0.18105 \, \frac{mg}{MHz} * A + 0.0438 \, \frac{mg}{MHz} * B + 1.470 * C. \qquad (5)$$

Als Standardabweichung zwischen den 15 Kalibrierdaten und den nach Kalibrierung errechneten Vorhersagen (Kreuzkalibrierung) erhält man $\sigma_{Kalibrierdaten}$ = 0.33 $mg$.

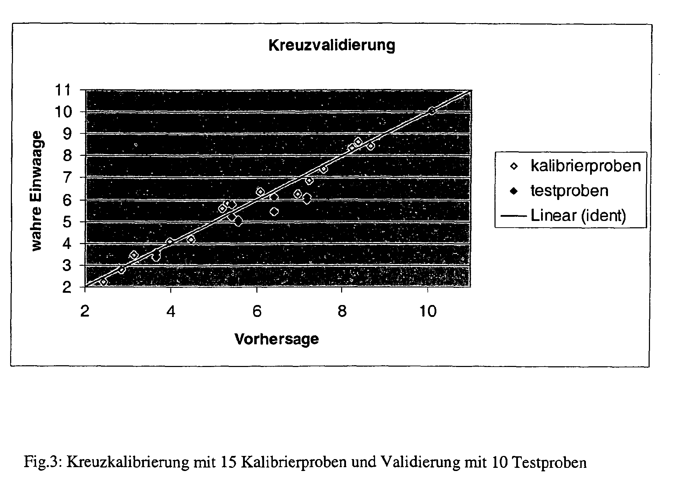

Nach der so durchgeführten Kalibrierung wurden die restlichen 10 Kapseln zum Test der Genauigkeit der vorgeschlagenen Verwiegemethode herangezogen. Hier liefert der Vergleich zwischen den wahren Einwaagewerten und den auf der Kalibrierung (5) basierenden Vorhersagen eine Standardabweichung von $\sigma_{Testproben}$ = 0.68 $mg$. Fig. 3 faßt alle Kalibrier- und Testdaten im Kreuzvalidierungsdiagramm zusammen.

Tab.1:  Meßwerte der 25 Kalibrier und Testproben

|  | Probe | Netto [mg] Analyse waage | Brutto [mg] Analyse waage | Tara [mg] Analyse waage | Mikrowelle F [MHz] | Mikrowelle B [MHz] | Brutto [mg] C [mg] | Vorhesage $m_N$ [mg] nach Gl. (5) |
|---|---|---|---|---|---|---|---|---|
| Kalibrierproben | 1 | 2.44 | 50.23 | 47.79 | 418.8594 | 103.9680936 | 50 | 2.24 |
|  | 2 | 2.85 | 51.06 | 48.21 | 424.9063 | 104.6123785 | 51.1 | 2.79 |
|  | 3 | 3.14 | 50.31 | 47.17 | 413.8125 | 100.6091802 | 50.3 | 3.45 |
|  | 4 | 3.97 | 52.04 | 48.07 | 425.9063 | 104.3166723 | 52.1 | 4.07 |
|  | 5 | 4.46 | 52.29 | 47.83 | 424.8594 | 102.5303456 | 52.1 | 4.18 |
|  | 6 | 5.19 | 52.46 | 47.27 | 416.9063 | 98.36373737 | 52.2 | 5.58 |
|  | 7 | 5.31 | 53.51 | 48.2 | 429.9063 | 103.8392956 | 53.8 | 5.82 |
|  | 8 | 6.08 | 54.36 | 48.28 | 430.875 | 102.979476 | 54.3 | 6.34 |
|  | 9 | 6.94 | 53.2 | 46.26 | 421.9375 | 101.2003059 | 53.2 | 6.27 |
|  | 10 | 7.56 | 54.7 | 47.14 | 427.9375 | 101.101946 | 54.7 | 7.38 |
|  | 11 | 8.23 | 56.18 | 47.95 | 434.9531 | 101.3368326 | 56.2 | 8.33 |
|  | 12 | 7.21 | 55.41 | 48.2 | 435.9453 | 102.809727 | 55.3 | 6.89 |
|  | 13 | 8.37 | 56.63 | 48.26 | 434.9375 | 101.9294894 | 56.4 | 8.65 |
|  | 14 | 8.67 | 56.97 | 48.3 | 440 | 101.7243854 | 56.9 | 8.46 |
|  | 15 | 10.08 | 58.3 | 48.22 | 440.9297 | 101.7071026 | 58.1 | 10.06 |
| Testproben | 16 | 7.16 | 56.27 | 49.11 | 451.0703 | 111.1018252 | 56.3 | 5.98 |
|  | 17 | 7.16 | 56.27 | 49.11 | 451.1563 | 111.1708615 | 56.4 | 6.12 |
|  | 18 | 5.56 | 53.51 | 47.95 | 432.0078 | 107.2317522 | 53.4 | 5.00 |
|  | 19 | 5.56 | 53.51 | 47.95 | 434.0625 | 107.6035326 | 53.7 | 5.09 |
|  | 20 | 6.38 | 54.16 | 47.78 | 433.0313 | 106.4290683 | 54.3 | 6.10 |
|  | 21 | 6.38 | 54.16 | 47.78 | 435.0625 | 106.8360262 | 54.1 | 5.46 |
|  | 22 | 5.42 | 52.74 | 47.32 | 424.0703 | 103.103133 | 52.7 | 5.23 |
|  | 23 | 5.42 | 52.74 | 47.32 | 426.125 | 103.9186604 | 53.3 | 5.77 |
|  | 24 | 3.65 | 51.65 | 48 | 426.0156 | 105.6539372 | 51.6 | 3.37 |
|  | 25 | 3.65 | 51.65 | 48 | 424.0938 | 105.4024385 | 51.5 | 3.56 |

**Patentansprüche**

1. Verfahren zur zerstörungsfreien Nettoverwiegung von Kapseln mit Hilfe von Mikrowellen, bei dem in einem ersten Verfahrensschritt durch einen Prozessor gesteuert in einem Mikrowellen-Generator eine elektromagnetische Strahlung variabler Frequenz erzeugt und einem als Resonator ausgebildeten Probenapplikator zugeführt wird, bei dem das aus dem Applikator austretende Mikrowellensignal einer Detektor-Diode zugeführt wird, aus deren Signalen über einen Analog/Digital-Wandler vom Rechner als primäre Meßgrößen b(0) und f(0) ermittelt werden, wobei b(0) die Halbwertsbreite bei der Resonanzfrequenz f(0) des mit einer Messprobe in Wirkverbindung stehenden Applikators ist, **dadurch gekennzeichnet, dass** mit dem Resonator eine Materialprobe so in Verbindung gebracht wird, dass das elektrische Feld des Resonators beim Übergang in das Material der Probe allgemein parallel zur Oberfläche verläuft und dass aus den primären Messgrössen b(0) und f(0) die Messsignale

$$F = f(L) - f(0) \text{ bzw. } B = b(0) - b(L)$$

erhalten werden, wobei F und B für die Mikrowellenmesssignale der Verstimmung und Verbreiterung der Resonanz stehen, mit $f(L)$ und b(L) gleich konstante materialabhängige Bezugsgrößen,
und bei dem in einem zweiten Schritt eine gravimetrische Bruttoverwiegung zur Bestimmung des Bruttowiegesignals

$$C = m_T + m_N,$$

in dem die Grösse $m_T$ für die Masse des leeren Gebindes und die Grösse $m_N$ für die zu bestimmende Nettomasse steht, durchgeführt wird, und abschließend aus den erhaltenen Messsignalen $F, B$ und $C$ die zu bestimmende Nettomasse $m_N$ gemäß nachfolgender Relation

$$m_N = \alpha A + \beta B + \gamma C + \delta$$

rechnerisch bestimmt wird, wobei die Koeffizienten $\alpha$, $\beta$, $\gamma$ und $\delta$ zuvor über Kalibrierung des Messsystems durch lineare Regression aus einer Meßreihe mit Referenzproben bekannter Einwaage bestimmt wurden.

2. Verfahren nach Anspruch 1, **dadurch gekennzeichnet, dass** befüllte Kapseln verwogen werden, deren Material ausgewählt ist aus der Gruppe bestehend aus Gelatine, Cellulosederivaten, Stärke, Stärkederivaten, Chitosan und synthetischen Kunststoffen.

3. Verfahren nach Anspruch 1 oder 2, **dadurch gekennzeichnet, dass** die Kapseln ein Pulver enthalten, welches einen Wirkstoff und ggebenenfalls einen pharmazeutisch verträglichen Hilfsstoff enthält.

4. Verfahren nach Anspruch 3, **dadurch gekennzeichnet, dass** der Wirkstoff ausgewählt ist aus der Gruppe der Anticholinergika, Betamimetica, Dopaminagonisten, Antiallergika, Leukotrien-Antagonisten und Corticosteroiden, sowie gegebenenfalls Wirkstoffkombinationen davon.

5. Verfahren nach einem der Ansprüche 1 bis 4, **dadurch gekennzeichnet, dass** die Meßsignale F und B mittels einer Vorrichung bestimmt werden, welche **gekennzeichnet ist durch** einen von einem Prozessor (2) digital abstimmbaren Mikrowellengenerator (3) mit variabler Frequenz, der mit einer Ankopplungssonde (5) verbunden ist, die in einem Applikator (4) zur Messung der Meßsignale $F$ und $B$ einer Probe (11) angeordnet ist, der eine weitere Ankopplungssonde (6) aufweist, die über einen Mikrowellen-Verstärker bzw. - Abschwächer (7) mit einer Detektor-Diode (8) verbunden ist, deren Signalausgang mit dem Prozessor (2) verbunden ist.

6. Verfahren nach einem der Ansprüche 1 bis 5, **dadurch gekennzeichnet, dass** zur Bestimmung des Bruttowiegesignals C eine gravimetrische Wiegezelle zum Einsatz gelangt, die das Gewicht mit einer Standardabweichung von +/- 0.1 mg bestimmt.

7. Verfahren nach einem der Ansprüche 1 bis 6, **dadurch gekennzeichnet, dass** es mittels einer Vorrichtung durchgeführt wird, welche einen Trichter (1) aufweist, aus dem die befüllten und verschlossenen Kapseln mittels einer Vereinzelungseinheit (2) seriell und/oder einzeln durch den Mikrowellenresonator (3) geführt werden, welche ferner eine sich daran anschließende Verteilereinheit (4) aufweist, durch welche der Transport der Kapseln parallelisiert wird und durch welche die Kapseln auf mehrere Spuren verteilt werden, in denen sich die gravimetrischen Wiegezellen (5) befinden, welche ferner eine computergestützte Datenerfassungseinheit (7) aufweist, die mit den Einheiten (3), (4) und (5) verbunden ist und für die richtige Zuordnung der Meßwerte zu den einzelnen Kapseln sorgt und die Auswertung durchführt und welche schließlich eine Weiche (6) aufweist, durch die Kapseln mit fehlgewichtiger Einwaage ausgeschieden werden.

8. Vorrichtung zur Durchführung der Nettoverwiegung von Kapseln, welche einen Trichter (1) aufweist, aus dem die befüllten und verschlossenen Kapseln mittels einer Vereinzelungseinheit (2) seriell und/oder einzeln durch den Mikrowellenresonator (3) geführt werden, welche ferner eine sich daran anschließende Verteilereinheit (4) aufweist, durch welche der Transport der Kapseln parallelisiert wird und durch welche die Kapseln auf mehrere Spuren verteilt werden, in denen sich die gravimetrischen Wiegezellen (5) befinden, welche ferner eine computergestützte Datenerfassungseinheit (7) aufweist, die mit den Einheiten (3), (4) und (5) verbunden ist und für die richtige Zuordnung der Meßwerte zu den einzelnen Kapseln sorgt und die Auswertung durchführt und welche schließlich eine Weiche (6) aufweist, durch die Kapseln mit fehlgewichtiger Einwaage ausgeschieden werden.

Fig 1: Skizze des Aufbaus zum Mikrowellenverfahren

Fig 2: Skizze zur Verbindung von Mikrowellendetektor und Wiegezellen

Fig.3: Kreuzkalibrierung mit 15 Kalibrierproben und Validierung mit 10 Testproben

**EP 1 484 586 A1**

| | Europäisches Patentamt | **EUROPÄISCHER RECHERCHENBERICHT** | Nummer der Anmeldung |
|---|---|---|---|
| | | | EP 03 01 2566 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A,D | DE 40 04 119 A (TEWS ELEKTRONIK DIPL ING MANFR) 14. August 1991 (1991-08-14) * das ganze Dokument * | 1,8 | G01G17/00 G01G9/00 G01N22/04 |
| A | US 2001/000946 A1 (NOACK ANDREAS ET AL) 10. Mai 2001 (2001-05-10) * Zusammenfassung; Abbildung 1 * | 1,8 | |
| A | WO 97 31244 A (CANE ARISTIDE ;IMA SPA (IT); AMAROLI SERGIO (IT); CONSOLI SALVATOR) 28. August 1997 (1997-08-28) * Zusammenfassung; Abbildungen 1,2 * | 1,8 | |
| A | US 5 515 740 A (GAMBERINI ERNESTO) 14. Mai 1996 (1996-05-14) * Zusammenfassung * | 1,8 | |

**RECHERCHIERTE SACHGEBIETE (Int.Cl.7)**

G01G
G01N

Der vorliegende Recherchenbericht wurde für alle Patentansprüche erstellt

| Recherchenort | Abschlußdatum der Recherche | Prüfer |
|---|---|---|
| DEN HAAG | 4. November 2003 | Ganci, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie,übereinstimmendes Dokument

EPO FORM 1503 03.82 (P04C03)

13

**ANHANG ZUM EUROPÄISCHEN RECHERCHENBERICHT**
**ÜBER DIE EUROPÄISCHE PATENTANMELDUNG NR.**   EP 03 01 2566

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-11-2003

| Im Recherchenbericht angeführtes Patentdokument | | Datum der Veröffentlichung | Mitglied(er) der Patentfamilie | | Datum der Veröffentlichung |
|---|---|---|---|---|---|
| DE 4004119 | A | 14-08-1991 | DE | 4004119 A1 | 14-08-1991 |
| | | | AT | 129343 T | 15-11-1995 |
| | | | DE | 59106709 D1 | 23-11-1995 |
| | | | WO | 9112518 A1 | 22-08-1991 |
| | | | EP | 0468023 A1 | 29-01-1992 |
| | | | ES | 2081471 T3 | 01-03-1996 |
| | | | US | 5397993 A | 14-03-1995 |
| US 2001000946 | A1 | 10-05-2001 | DE | 19734978 A1 | 18-02-1999 |
| | | | US | 6163158 A | 19-12-2000 |
| | | | DE | 19705260 A1 | 21-08-1997 |
| | | | EP | 0791823 A2 | 27-08-1997 |
| | | | JP | 9325123 A | 16-12-1997 |
| | | | CN | 1213080 A | 07-04-1999 |
| | | | EP | 0902277 A1 | 17-03-1999 |
| | | | JP | 11108856 A | 23-04-1999 |
| WO 9731244 | A | 28-08-1997 | IT | BO960077 A1 | 21-08-1997 |
| | | | CA | 2247073 A1 | 28-08-1997 |
| | | | CN | 1200807 A ,B | 02-12-1998 |
| | | | DE | 69706967 D1 | 31-10-2001 |
| | | | DE | 69706967 T2 | 04-04-2002 |
| | | | WO | 9731244 A1 | 28-08-1997 |
| | | | EP | 0886765 A1 | 30-12-1998 |
| | | | ES | 2161438 T3 | 01-12-2001 |
| | | | JP | 2000505405 T | 09-05-2000 |
| | | | US | 6114636 A | 05-09-2000 |
| US 5515740 | A | 14-05-1996 | IT | 1258025 B | 20-02-1996 |
| | | | CH | 689553 A5 | 15-06-1999 |
| | | | DE | 4325569 A1 | 03-02-1994 |
| | | | FR | 2694264 A1 | 04-02-1994 |
| | | | GB | 2269354 A ,B | 09-02-1994 |
| | | | JP | 3419826 B2 | 23-06-2003 |
| | | | JP | 7284519 A | 31-10-1995 |

EPO FORM P0461

Für nähere Einzelheiten zu diesem Anhang : siehe Amtsblatt des Europäischen Patentamts, Nr.12/82